# EUROPEAN PATENT SPECIFICATION

(11) **EP 2 854 993 B1**
(45) Date of publication and mention of the grant of the patent: **26.12.2018**
(21) Application number: 13727781.0
(22) Date of filing: 29.05.2013
(51) Int. Cl.: B01D 53/14, B01D 53/52, B01D 53/78, C01B 17/16, C07C 217/00

(54) **A METHOD OF IMPROVING A PROCESS FOR THE SELECTIVE ABSORPTION OF HYDROGEN SULFIDE**
METHODE ZUR VERBESSERUNG EINES PROZESSES ZUR SELEKTIVEN ABSORPTION VON SCHWEFELWASSERSTOFF
MÉTHODE POUR L'AMÉLIORATION D'UN PROCÉDÉ POUR L'ABSOPRTION SÉLECTIVE DE SULFURE D'HYDROGÈNE

(30) Priority: 31.05.2012 US 201261653936 P
(43) Date of publication of application: 08.04.2015
(73) Proprietor: Shell Internationale Research Maatschappij B.V., 2596 HR The Hague (NL); Huntsman Petrochemical Corporation, The Woodlands, TX 77380 (US)
(72) Inventor: CRITCHFIELD, James Edward, Austen, Texas 78731 (US); VALENZUELA, Diego Patricio, NL-1031HW Amsterdam (NL); WILSON, Loren Clark, Katy, Texas 77494 (US); ZHOU, Jingjun, The Woodlands, Texas 77382 (US)
(74) Representative: Shell Legal Services IP
(86) International application number: PCT/US2013/043115
(87) International publication number: WO 2013/181252

(56) References cited:
- WO-A1-2012/074807
- US-A- 3 989 811
- US-A- 4 894 178

## Description

This invention relates to an absorbent composition that is useful in the selective removal of hydrogen sulfide from gas streams containing hydrogen sulfide and carbon dioxide, including use of the absorbent composition, and a method of improving a process for the selective removal of hydrogen sulfide from a gas stream containing hydrogen sulfide and carbon dioxide.

The use of certain amine compounds and solutions for the separation of acidic gases such as CO₂, H₂S, CS₂, HCN, and COS from gaseous mixtures is known in the art of gas treating. One early method of separating acidic gases from gaseous mixtures is disclosed in U. S. Pat. No. 3,347,621. The process disclosed in this patent uses a liquid absorbent that comprises an alkanolamine and a sulfone that is contacted with a gas mixture containing acidic gas components. Examples of other early patents that disclose the use of solutions of alkanolamine and sulfone in the treatment of gaseous mixtures that contain significant concentrations of H₂S, CO₂ and COS include U.S. Pat. No. 3,965,244 and U.S. Pat. No. 3,989,811.

In a later patent, U.S. Pat. No. 4,894,178, there is disclosed the use of a mixture of two severely hindered amines in the selective removal of H₂S from gas mixtures that contain both H₂S and CO₂. One example presented of a mixture of the two severely hindered amines includes bis(tertiarybutyl aminoethoxy)-ethane (BTEE) and ethoxyethoxyethanol-tertiarybutyl amine (EEETB). This mixture is obtained by the one-step catalytic tertiarybutylamination of triethylene glycol to yield a first amine, e.g. BTEE, and a second amine, e.g. EEETB, having a weight ratio of the first amine to second amine in the range of from 0.43:1 to 2.3:1.

The '178 patent indicates that one problem with the use of aqueous solutions of BTEE is that they suffer from phase separation under regeneration conditions. The '178 patent further indicates that EEETB can be used for the selective removal of H₂S in the presence of CO₂ and that a mixture of BTEE and EEETB not only provides for better selectivity and higher capacity for H₂S than EEETB alone, it also does not phase separate under regeneration conditions as do aqueous solutions of BTEE.

Prior to the use of the amine mixture that is disclosed in the '178 patent, it is taught that the amine mixture may be contained in a liquid medium such as water, an organic solvent and mixtures thereof. The preferred liquid medium comprises water, but possible other suitable solvents include the physical absorbents described in U.S. Pat. No. 4,112,051. Sulfones, such as sulfolane, are among the suitable physical absorbents. The liquid medium can be a mixture of water and organic solvent and is typically present with the absorbent in an amount in the range of from 0.1 to 5 moles per liter, preferably from 0.5 to 3 moles per liter, of the total absorbent composition. It is not clear, however, what mole units of which the '178 patent is referring.

U.S. Pat. No. 4,961,873 discloses an absorbent composition that comprises a mixture of two severely hindered amines similar to the mixture disclosed in U.S. Pat. No. 4,894,178 with a weight ratio of a first amine to a second amine being in the range of from 0.43:1 to 2.3:1, an amine salt and/or a severely hindered aminoacid. The severely hindered amine mixture and severely hindered amine salt and/or aminoacid additives are dissolved in a liquid medium. The amine mixture and additive of the absorbent composition before it is contained in the liquid medium comprises from 5 to 70 wt % amine mixture, from about 5 to 40 wt % additive, and the balance being water with the weight percent being based on the weight of the total liquid absorbent composition.

As in the '178 patent, the '873 patent teaches that, prior to the use of the liquid absorbent composition that includes the severely hindered amine mixture, it may be contained in a liquid medium such as water, an organic solvent and mixtures thereof. The preferred liquid medium comprises water, but possible other suitable solvents include the physical absorbents described in U.S. Pat. No. 4,112,051. Sulfones, such as sulfolane, are among the suitable physical absorbents. The liquid medium can be a mixture of water and organic solvent and is typically present with the absorbent in an amount in the range of from 0.1 to 5 moles per liter, preferably from 0.5 to 3 moles per liter, of the total absorbent composition. It is not clear, however, what mole units of which the '873 patent is referring.

In the art of gas treating there are ongoing efforts to find new and improved absorbent compositions useful in the removal of acidic gaseous components contained in normally gaseous hydrocarbon streams. For some gas treating applications, it can be desirable to treat gas mixtures that contain both CO₂ and H₂S so as to selectively remove from such gas mixtures the H₂S while minimizing the removal of the CO₂. Sometimes, a gas stream to be treated for the selective removal of H₂S may already have a low concentration of H₂S, relative to its CO₂ concentration, that needs to be further reduced. One example of such process gas streams to be treated includes Claus tail gases. These tail gas streams typically have high concentrations of carbon dioxide but relatively low concentrations of hydrogen sulfide, and it is often desirable to selectively remove the H₂S to thereby provide a concentrated stream of H₂S for introduction to a Claus sulfur unit.

Accordingly, provided is an absorbent composition that is useful in the selective removal of hydrogen sulfide from gas mixtures containing hydrogen sulfide and carbon dioxide. The absorbent composition comprises: (a) from 75 wt. % to 92 wt.%, based on the total weight of said absorbent composition, of an aqueous solvent; and (b) from 8 wt. % to 25 wt. %, based on the total weight of said absorbent composition, of an organic co-solvent, wherein said aqueous solvent comprises from 20 wt. % to 70 wt. %, based on the total weight of said aqueous solvent, of an amination reaction product of a polydispersed polyethylene glycol (PEG) mixture having an average molecular weight that is in the range of from 180 to 1000 and t-butylamine, wherein said amination reaction product further comprises at least a first sterically hindered amine and a second sterically hindered amine, and from 30 wt. % to 80 wt. % water, based on the total weight of said aqueous solvent, and wherein said organic co-solvent is selected from the group consisting of sulfones, sulfone derivatives, and sulfoxides.

Also provided is a method according to claim 1 for improving a process which utilizes the amine absorbent for the selective removal of hydrogen sulfide from a gas stream that comprises hydrogen sulfide and carbon dioxide.
FIG. 1 is a schematic flow diagram illustrating an absorption-regeneration system for treating gaseous streams that contain H₂S and CO₂ to selectively remove H₂S therefrom.
FIG. 2 presents plots of the measured rate ratios (H2S absorption rate/CO₂ absorption rate) as a function of H₂S in the treated gas for the amine mixture of the invention and for MDEA.
FIG. 3 presents plots of the measured H₂S concentration in a treated gas as a function of the CO₂ contained in the gas to be treated provided by the amine mixture of the invention and MDEA.
FIG. 4 presents plots of the percentage of the total CO₂ contained in a feed gas stream that is absorbed either by the amine mixture of the invention or by MDEA as a function of the concentration CO₂ in the feed gas stream.

The absorption composition of the invention is particularly useful in the selective absorption of hydrogen sulfide from gaseous mixtures that comprise hydrogen sulfide and carbon dioxide. The composition further may have application in the absorption removal of other acidic gases in addition to hydrogen sulfide (H₂S).
The gas streams that are to be treated by use of the composition of the invention may be obtained from a wide variety of sources of gaseous mixtures. The gaseous mixtures can include the hydrocarbon-containing gases generated by processes involving pyrolysis of bituminous sands and hydrocarbon-containing gases produced or generated by refinery coker and cracking units and by other crude petroleum refinery operations. Natural gas streams having concentrations of acidic compounds, such as the compounds previously mentioned, can also be treated with the composition of the invention.

Moreover, the composition may be used to treat gas streams that contain very low concentrations of hydrocarbons and, even, no material concentration or substantially no concentration of hydrocarbons or otherwise having a material absence of hydrocarbons. One example of such a gas stream having a very low hydrocarbon concentration, if any, is a Claus unit tail gas stream.

Due to its high selectivity in the absorption of H₂S relative to CO₂ and to its high H₂S loading capacity, the absorbent composition of the invention is especially useful in the treatment of Claus tail gas streams. Claus tail gas streams typically have small concentrations of H₂S relative to their concentrations of carbon dioxide, but the H₂S concentrations tend to be too high to permit the streams from being combusted or released into the atmosphere. Therefore, it often is desirable to remove a substantial portion of the H₂S from the tail gas stream and to use the removed H₂S as a recycle feed to the Claus unit. However, it typically is not desirable to recycle CO₂ with the recovered H₂S to the Claus unit; because, the CO₂ loads up the unit by passing through it unchanged.

Claus unit tail gas streams typically can have an H₂S concentration that is in the range of from or about 0.2 vol. % (2,000 ppmv) to or about 4 vol. % (40,000 ppmv). More specifically, the H₂S concentration can be in the range of from 4,000 ppmv to 15,000 ppmv, and, even, from 6,000 ppmv to 12,000 ppmv.

The CO₂ concentration of the tail gas stream can sometimes range upwardly to 90 vol. % of the gas stream, depending upon the particular combustion gas that is used in the thermal step of the Claus unit. For instance, if a pure oxygen combustion gas is used in a thermal step of the Claus unit to burn the H₂S, there will be very little nitrogen in the tail gas and a very high concentration of CO₂. But, when air is used as the combustion gas, then the CO₂ concentration in the tail gas will be much lower and the N₂ concentration will be a significant component of the tail gas. Generally, the CO₂ concentration in the tail gas is considerably higher than its H₂S concentration, and the CO₂ concentration of the tail gas can be in the range of from 1 vol. % (10,000 ppmv) to 60 vol. %. More particularly, the CO₂ concentration is in the range of from 2 vol. % to 50 vol. % or from 3 vol. % to 40 vol. %.

In the typical case in which air is the combustion gas of the Claus unit thermal step, the tail gas stream includes a major portion that is molecular nitrogen (N₂), which typically is in the concentration range of from 40 to 80 vol. %.

The absorbent composition provides for a treated tail gas having an exceptionally low H₂S concentration of less than 100 volume parts per million (ppmv), but, more specifically, the H₂S concentration of the treated tail gas is less than 50 ppmv. It preferred for the concentration of H₂S in the treated tail gas to be less than 25 ppmv, and more preferred, it is less than 10 ppmv. A practical lower limit for the H₂S concentration of the treated tail gas is 1 ppmv, and, more typically, no lower than about 5 ppmv, but it is understood that it is generally desired for the treated tail gas to have the lowest concentration of H₂S as is possible.

An essential component of the absorbent composition of the invention is the mixture of amine compounds that is included as one of the components of the aqueous solvent of the absorbent composition. It is believed that the particular mixture of amines and its properties contribute to some of the special selectivity and absorption characteristics of the inventive absorbent composition.

The amine mixture component of the aqueous solvent and absorbent composition is an amination reaction product. The amination reaction product is prepared by the catalytic reaction, under suitable reaction conditions as more fully described elsewhere herein, of an amine compound that is tert-butylamine, having the formula (CH₃)₃CNH₂, with polyethylene glycol, as represented by the following formula: HOCH₂(CH₂OCH₂)ₙCH₂OH, wherein n is an integer selected from values in the range of from 1 to 24.

One of the attributes of the amine mixture, or amination reaction product, results from the characteristics of the polyethylene glycol (also referred to herein as "PEG") reactant that is used in the preparation of the amine mixture. The PEG reactant does not consist of only a single PEG molecule, but it comprises more than a single PEG molecule.

The PEG reactant used in the preparation of the amination reaction product is a mixture comprising two or more or a distribution of different PEG molecules having the aforementioned formula, wherein, for each of the individual PEG molecules, the integer n is a different value. Therefore, the amine mixture is not a reaction product of tert-butylamine and a single molecule of PEG, for example, triethylene glycol, but, instead, it is a reaction product of tert-butylamine with a distribution of PEG molecular compounds.

The mixture of PEG compounds used in preparing the amination reaction product includes two or more different PEG compounds having the aforementioned formula, wherein n is an integer selected from values in the range of from 1 to 24. It is preferred for the PEG mixture to comprise two or more molecules of the aforementioned formula, wherein the integer n is selected from the range of integers from 2 to 20, and, preferably from the range of integers from 2 to 18, and, most preferably, from the range of integers from 3 to 15.

The mixture of PEG compounds used as the reactant has an average molecular weight in the range of from 180 to 1,000. Thus, the combination of individual PEG molecules and their relative concentrations in the mixture of PEG compounds used as a reactant in the preparation of the amination reaction product are such as to provide a mixture of PEG compounds having the indicated average molecular weight in the range of from 180 to 1,000. It is preferred for the PEG mixture used as a reactant in the preparation of the amination reaction product to have an average molecular weight that is in the range of from or about 180 to or about 400, and, more preferably, the average molecular weight is in the range of from 200 to 300.

The average molecular weight as used herein is the number average molecular weight as determined by measuring the molecular weight of each PEG molecule of the PEG mixture, summing the weights, and then dividing by the number of PEG molecules of the PEG mixture.

The amination reaction for preparing the amine mixture of the invention is carried out by contacting the reactants, i.e., tert-butylamine, PEG mixture, and hydrogen, with the amination catalyst of the invention under suitable amination reaction conditions to yield the amine mixture, i.e., the amination reaction product.

The selection of an amination catalyst for use in this catalytic reaction is important in providing an amine mixture having the properties and characteristics required of the invention. It is a combination of the characteristics and properties of the PEG reactant along with those of the amination catalyst used in the amination reaction that provides the unique amine mixture of the invention. Therefore, the composition and other characteristics of the amination catalyst can be an important if not a critical aspect of the invention.

The amination catalyst that is used in the preparation of the amine mixture contains catalytically active metal components, including, a nickel (Ni) component, a copper (Cu) component and either a zirconium (Zr) component or a chromium (Cr) component, or both, and, optionally, but preferably, a tin (Sn) component. It may be desirable in some instances for the amination catalyst to have a material absence of or substantial absence of or absence of such a metal as cobalt (Co), or tungsten (W) or molybdenum (Mo), or rhenium (Re) or any combination of one or more thereof. In certain other embodiments of the amination catalyst, it may have a material absence or substantial absence or absence of either zirconium or chromium, but not both metal components.

Possible amination catalyst compositions that may be used in preparing the amine mixture are disclosed and described in U.S. Patent No. 4,152,353; U.S. Patent No. 6,057,442; U.S. Patent No. 7,196,033; and U. S. Patent No. 7,683,007.

The amination catalyst comprises: from 40 to 90 wt. % nickel; from 4 to 40 wt. % copper; and from 1 to 50 wt. % of either zirconium or chromium, or a combination of both zirconium and chromium. The amination catalyst may further comprise, and preferably does comprise, from 0.2 to 20 wt. % tin.

The amination catalyst may be prepared by any of a variety of methods known to those skilled in the art to make a catalyst of the aforedescribed composition; provided, that such a catalyst is used in preparing the amine mixture of the invention. One example of a method of preparing the amination catalyst is by peptizing powdered mixtures of hydroxides, carbonates, oxides, or other salts of the metal (nickel, copper, zirconium, chromium, and tin) components with water in proportions so as to provide a composition as defined herein, and subsequently extruding and heat-treating the resulting composition.

The amination reaction may be conducted with any suitable reactor arrangement or configuration and under any suitable reaction conditions that provide for the desired amination reaction product. Examples of possible reactors for carrying out the amination reaction include fixed-bed reactors, fluid-bed reactors, continuous stirred reactors, and batch reactors.

The first sterically hindered amine is selected from the group of amine compounds having the following formula:

(CH₃)₃CNH(CH₂CH₂O)ₓCH₂CH₂NHC(CH₃)₃,

wherein x is an integer in the range of from 3 to 14.

The second sterically hindered amine is selected from the group of amine compounds having the following formula:

(CH₃)₃CNH(CH₂CH₂O)ₓCH₂CH₂OH,

wherein x is an integer in the range of from 3 to 14.

The amine mixture of the absorbent composition has a weight ratio of the first sterically hindered amine to the second sterically hindered amine in the range of from 3:1 to 6:1.

The absorbent composition comprises the amine mixture, as described above, in combination with water to thereby provide or form an aqueous solvent that is a component of the absorbent composition.

The amine mixture component of the aqueous solvent is present in an amount in the range of from 20 wt. % to 70 wt. % and the water component is generally present in an amount in the range of from 30 wt. % to 80 wt. %. The weight percent values recited for these components are based on the total weight of the aqueous solvent or the amine mixture plus water.

It is preferred for the aqueous solvent to comprise from 25 wt. % to 65 wt. % amine mixture, or from 35 wt. % to 55 wt. % amine mixture. It is more preferred for the amine mixture to be present in the aqueous solvent in the range of from 40 wt. % to 50 wt. %.

The water content of the aqueous solvent can be in the preferred range of from 35 wt. % to 75 wt. %, or from 45 wt. % to 65 wt. %, and, more preferred, the water content is from 50 wt. % to 60 wt. %.

It has been discovered that one problem with the use of the amine mixture or the aqueous solvent in the absorption treatment of gas mixtures is that it separates into several phases at temperatures falling within the range of regeneration temperatures for the amine mixture or aqueous solvent. The amine mixture or aqueous solvent can be used in processes for the treatment gas streams having concentrations of acidic gases and the removal of gases therefrom. These processes may use systems for treating the gas streams, wherein the systems include a contacting column and a regenerator system that includes a regenerator column which is usually equipped with a reboiler.

The contacting column of the treating system provides means for contacting a lean amine mixture or a lean aqueous solvent with a gas stream or mixture, having a concentration of one or more acidic gas components, such as H₂S, to yield a treated gas stream and an H₂S rich amine mixture or H₂S rich aqueous solvent. The regenerator system provides means for receiving and regenerating the H₂S rich amine mixture or H₂S rich aqueous solvent to yield the H₂S lean amine mixture or H₂S lean aqueous solvent for introduction into and use within the contacting column.

A regenerator system typically includes a regenerator column that provides means for separating the absorbed acid gas components from the H₂S rich amine mixture or H₂S rich aqueous solvent. Operatively connected or associated with the regenerator column is a reboiler that provides means for introducing heat into the amine mixture or aqueous solvent and to otherwise provide heat energy for the operation of the regenerator system. In the operation of the regeneration system, the regeneration temperature can vary depending upon the operating pressure of the regenerator and the composition of the amine mixture or aqueous solvent being regenerated.

Typically, the regeneration temperature is within the range of from 80 °C to 150 °C. A more specific regeneration temperature is in the range of from 85 °C to 140 °C, and, especially more specific, the regeneration temperature is in the range of from 90 °C to 130 °C.

As mentioned earlier, it has been discovered that the amine mixture and aqueous solvent compositions tend to separate into two or more liquid phases at certain elevated temperature conditions. Particularly, the amine mixture or aqueous solvent is thought to phase separate under the conditions at which the aforementioned regenerator system is operated. This phase separation phenomenon is unexpected; since, certain teachings within the prior art indicate that various mixtures of severely hindered amines that are different from the amine mixtures defined herein do not phase separate under conditions of regeneration. The phase separation is not desired and may pose certain operating problems or, at least, contribute to higher cost of operation of gas treating systems. It has been found, however, that certain problems associated with phase separation that occur with the amine mixture and aqueous solvent may be solved by the use and application of an organic co-solvent. Thus, the absorbent composition beyond the amine mixture and aqueous solvent as described herein is provided by incorporating an amount of organic co-solvent with the amine mixture or aqueous solvent at a concentration that is effective to promote the miscibility of the individual components of the amine mixture or of the aqueous solvent.

The specific organic co-solvent is selected from the group of organic compounds consisting of sulfones, sulfone derivatives, and sulfoxides. These compounds are defined and described in great detail in U.S. Patent No. 4,112,051; U.S. Patent No. 3,347,621; and U.S Patent No. 3,989,811. The preferred organic co-solvent is a sulfone, and, among the sulfones, a substituted or unsubstituted cyclotetramethylene sulfone (sulfolane) is the more preferred. The most preferred sulfone is sulfolane.

The sulfone compounds of the inventive absorption composition have the general formula: 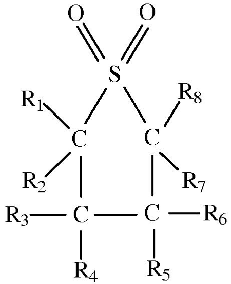 wherein at least four of the R substituents are hydrogen radicals and any remaining Rs being alkyl groups having from 1 to 4 carbon atoms. It is preferred that no more than two alkyl substituents are appended to the tetramethylene sulfone ring.

Suitable sulfone derivatives include 2-methyl tetramethylene sulfone; 3-methyl tetra methylene sulfone; 2,3-dimethyl tetramethylene sulfone; 2,4-dimethyl tetramethylene sulfone; 3,4- dimethyl tetramethylene sulfone; 2,5-dimethyl tetramethylene sulfone; 3-ethyl tetramethylene sulfone; 2-methyl-5-propyl tetramethylene sulfone as well as their analogues and homologues.

An embodiment of the absorbent composition of the invention, therefore, can include a combination of the organic co-solvent and the aqueous solvent which, as described herein, includes the amine mixture and water.

The aqueous solvent component of the absorbent composition can be present in an amount in the range of from or about 75 wt. % to or about 92 wt. %, with the weight percent being based on the total weight of the absorbent composition (i.e. the aqueous solvent plus organic co-solvent).

As for the organic co-solvent component of the absorbent composition, the amount present in the absorbent composition should be such that it is effective to promote the miscibility of the components of the aqueous solvent especially at the elevated temperatures at which such components are at least partially immiscible. This concentration level of organic co-solvent can be in the range of from or about 8 wt. % to or about 25 wt. %, with the weight percent being based on the total weight of the absorbent composition.

The preferred concentration of organic co-solvent in the absorbent composition is in the range of from 8 wt. % to 15 wt. %.

The absorbent composition of the invention is useful in the treatment of gaseous mixtures comprising acidic gas components by the absorption removal of the acidic gas components therefrom. The absorbent composition is particularly useful in the selective removal of H₂S from gaseous streams that comprise both H₂S and CO₂. This is accomplished by contacting, under absorption conditions, the gaseous stream with the absorbent composition typically by utilizing an absorber or contacting vessel. The absorber is operated under suitable contacting or absorption process conditions for the selective absorption and removal of the H₂S from the gaseous stream.

Generally, the absorption step is conducted by feeding the gaseous stream into the lower portion of an elongated contacting or absorption vessel that defines a contacting or absorption zone. The contacting or absorption zone is typically equipped with contacting trays or packing or any other suitable means for promoting the contacting of the absorbent composition with the gaseous stream.

The absorbent composition that is lean in H₂S is introduced into upper portion of the elongated vessel and flows countercurrently with the gaseous stream that is introduced into the lower portion of the vessel. As the absorbent composition passes through the contacting vessel it is contacted with the gaseous stream and selectively removes H₂S from the gaseous stream. A treated gas stream having a reduced concentration of H₂S is yielded from the upper end of the vessel and the absorbent composition rich in H₂S is yielded from the bottom portion of the vessel.

The inlet temperature of the H₂S lean absorbent composition, and, thus, the contacting temperature of the H₂S lean absorbent composition with the gaseous mixture, typically is in the range of from or about 5 °C to or about 50 °C and, more typically, from 10 °C to 45 °C.

The operating pressure of the absorption vessel is typically in the range of from 34.47 kPa abs. to 13789.51 kPa abs. (5 psia to 2,000 psia), but, more suitably, it is in the range of from 137.89 to 10342.14 kPa abs. (20 to 1,500 psia).

The H₂S rich absorption composition from the absorber may be regenerated by any suitable means or method for providing the H₂S lean absorbent composition for use in the absorber contactor. In one typical regeneration step, the H₂S rich absorption composition is introduced into a regenerator vessel of a regeneration system for receiving and regenerating the H₂S rich absorption composition to yield the H₂S lean absorbent composition. The regenerator vessel defines a regeneration zone into which the H₂S rich absorption composition is introduced and the regenerator vessel provides means for regenerating the H₂S rich absorption composition by stripping the absorbed H₂S therefrom.

The regenerator is typically equipped with a reboiler that provides heat energy for stripping the H₂S and other acidic gas components from the H₂S rich absorption composition. The regeneration temperature is typically in the range of from or about 80 °C to or about 170 °C, and, more typically, from 85 °C to 140 °C.

The regeneration pressure is typically in the range of from 6.89 kPa abs. to 344.74 kPa abs. (1 psia to 50 psia), more typically, from 103.42 kPa abs. to 275.79 kPa abs. (15 psia to 40 psia), and, most typically, from 137.89 kPa kbs. to 241.32 kPa abs. (20 psia to 35 psia).

Provided is a method of improving a process for the selective removal of hydrogen sulfide from gas streams that comprise hydrogen sulfide and carbon dioxide. In these processes, certain conventional absorption and regeneration process systems are used for the treatment of gas streams containing acidic gas components. These process systems typically contain an inventory of an amine absorbent that includes an H₂S lean amine and an H₂S rich amine. The process system further includes a contacting column for contacting the H₂S lean absorbent with the gas stream to yield a treated gas stream and the H₂S rich absorbent and a regenerator for receiving and regenerating the H₂S rich absorbent from the contacting column to yield the H₂S lean absorbent that is introduced into the contacting column. This process is improved either by providing or replacing the amine absorbent with the absorbent composition of the invention.

Thus, a method is provided for improving a process which utilizes an amine absorbent composition for the selective removal of hydrogen sulfide form a gas stream containing hydrogen sulfide and carbon dioxide. In this method, the absorbent composition of the invention, as described in detail herein, is provided and utilized in the absorption treatment of the gas stream in the manner and by the methods as more fully described elsewhere herein.

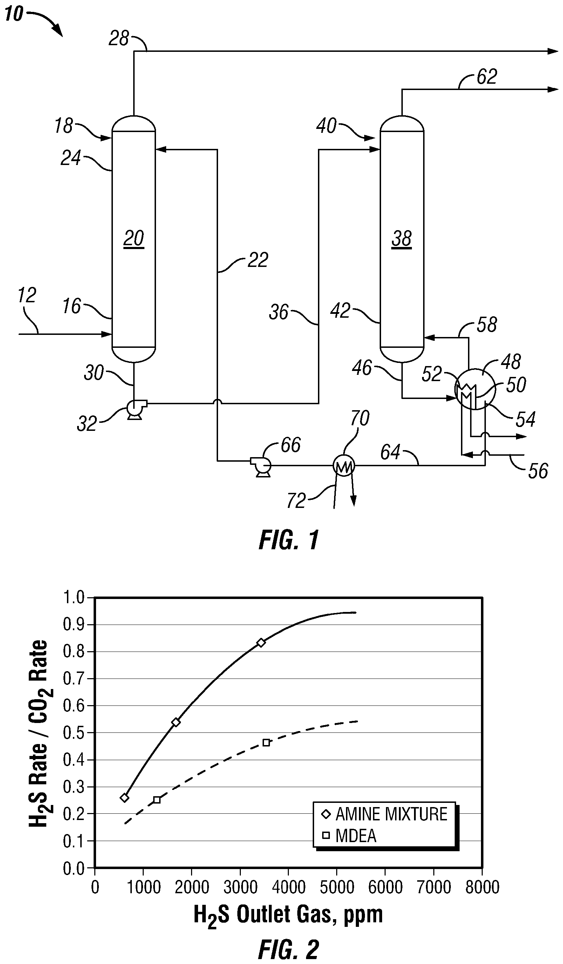

Reference is now made to FIG. 1, which is a schematic flow representation of absorption-regeneration system 10 for treating gaseous streams that contain hydrogen sulfide and carbon dioxide, particularly, to selectively remove hydrogen sulfide from the gaseous stream and to yield a treated gas having a reduced hydrogen sulfide concentration. The gaseous stream, comprising H₂S and CO₂, that is to be treated passes by way of conduit 12 and is introduced, preferably, into the lower portion 16 of contactor/absorber 18.

Contactor/absorber 18 defines a contacting/absorption zone 20, wherein an H₂S lean absorbent composition of the invention is contacted with the gaseous stream under absorption conditions for providing the selective absorption of H₂S from the gaseous stream by the H₂S lean absorbent composition.

The H₂S lean absorbent composition passes by way of conduit 22 and is introduced, preferably, into contacting/absorption zone 20 of the upper portion 24 of contactor/absorber 18. The H₂S lean absorbent composition passes through contacting/absorption zone 20 wherein it is contacted in a countercurrent fashion with the gaseous stream also passing through contacting/absorption zone 20 to thereby selectively absorb the H₂S contained in the gaseous stream.

A treated gas stream, having a reduced concentration of H₂S, is yielded and withdrawn from contacting/absorption zone 20 and passes by way of conduit 28 to downstream. An H₂S rich absorbent composition is yielded and withdrawn from contacting/absorption zone 20 and passes by way of conduit 30 to pump 32 that defines a pumping zone and provides means for imparting pressure energy into and conveying the H₂S rich absorbent composition.
The H₂S rich absorbent composition passes by way of conduit 36 from pump 32 for introduction into regeneration zone 38, which is defined by regenerator 40. Regenerator 40 provides means for receiving and regenerating the H₂S rich absorbent composition to yield the H₂S lean absorbent composition and off-gas, comprising H₂S. Typically, the H₂S rich absorbent composition flows downwardly through regeneration zone 38 and exits the lower portion 42 of regenerator 40 through conduit 46.

A bottoms stream then passes from regeneration zone 38 to reboiler 48. Reboiler 48 defines a reboiling zone (not labeled) wherein heat energy is introduced for use in vaporizing a portion, principally water, of the bottoms stream and for driving the H₂S therefrom. Any suitable type of reboiler known to those skilled in the art may be used as reboiler 48, but the one represented is a kettle-type reboiler having an internal weir 50 that defines within reboiler 48 a liquid volume section 52 on one side of internal weir 50 and reboiler sump section 54 on the other side of internal weir 50. Heat energy is introduced into the liquid volume section 52 by passing through steam coil 56. Vapor, which can comprise H₂S and water, passes from reboiler 48 by way of conduit 58 to lower portion 42 of regenerator 40.

An off-gas stream, comprising H₂S, is yielded and passes from regenerator 40 by way of conduit 62. Hot H₂S lean absorbent composition is withdrawn from reboiler sump section 54 and passes therefrom by way of conduit 64 to pump 66. Interposed in conduit 64 is heat exchanger 70. Heat exchanger 70 defines a heat transfer zone and provides means for cooling the hot H₂S lean absorbent composition, preferably by indirect heat exchange with cooling water passing through cooling tubes 72 to thereby provide the cooled H₂S lean absorbent composition that passes to pump 66. Pump 66 provides for conveying the cooled H₂S lean absorbent composition by way of conduit 22 for introduction into and reuse in contacting/absorption zone 20 of contactor/absorber 18.

The following examples are provided to illustrate certain embodiments of the invention, but they should not be considered as limiting the invention in any respect.

### Example 1

This Example 1 describes the experiment for testing certain phase separation characteristics of various absorbent compositions and the effect of the organic co-solvent (sulfolane) on phase separation at elevated temperatures. Presented in Table 1 are the results of the testing.

The amine mixture used in preparing the compositions for this Example 1 and the other examples herein was an amination reaction product prepared by the catalytic reaction of tert-butylamine in the presence of a nickel amination catalyst, as described herein, at a reaction temperature of 200 °C and a reaction pressure of 13789.51 kPa gauge (2,000 psig) with a polydispersed polyethylene glycol (PEG) mixture of an average molecular weight in the range of from 180 to 1000, and, in particular, a PEG mixture with an average molecular weight of about 240.

Various solutions of the amine mixture, water and the organic co-solvent, sulfolane, were prepared and placed in sealed glass tubes. All of the solutions were clear and exhibited a single phase at room temperature. The sealed glass tubes were placed in a silicone oil bath and heated. As the temperature of the solutions increased, many became cloudy and exhibited phase separation at various temperatures.

Presented in Table 1 are the compositions of the various solutions or absorbent compositions that were tested and the temperatures at which separation into several liquid phases were observed for each. It is desirable for there to be no liquid-liquid phase separation of the components at a temperature of at least greater than 120 °C.

**Table 1. Absorbent compositions and temperatures at which phasing occurs.**

| Sample No. | Amine Mixture | Water | Sulfolane | Temperature at which Phasing was observed |
|---|---|---|---|---|
| | (wt. %) | (wt. %) | (wt. %) | (°C) |
| Comp. 1 | 40 | 60 | 0 | 120 |
| Comp.2 | 29.9 | 70.1 | 0 | 110 |
| Comp.3 | 20 | 80 | 0 | 100 |
| Comp.4 | 11.9 | 88.1 | 0 | 105 |
| 5 | 34.8 | 52.3 | 12.9 | > 120 |
| 6 | 26 | 60.7 | 13.3 | > 120 |
| 7 | 17.1 | 68.6 | 14.3 | > 120 |
| 8 | 10.2 | 75.2 | 14.6 | > 120 |
| Comp.9 | 37.9 | 56.8 | 5.3 | > 120 |
| 10 | 36.3 | 54.5 | 9.2 | > 120 |
| Comp.11 | 19 | 76 | 5 | 114.6 |
| 12 | 18.1 | 72.9 | 9 | > 120 |

This Example shows that the aqueous solvent (i.e., amine mixture and water) phase separates, over a range of elevated temperatures. This Example also demonstrates that liquid phase separation occurs over a wide range of concentrations of the amine mixture component of the absorbent composition (solution). The data show that solutions having a concentration of the amine mixture component of around 20 wt. % require more co-solvent in order to maintain a single liquid phase. This is shown by the results for sample numbers 3 (comparative), 11 (comparative) and 12. At this concentration level for the amine mixture component, the amount of co-solvent required to prevent the phase separation or maintain the single phase at the elevated temperatures is in the range of from 5 wt. % to 9 wt. %.

### Example 2

This Example describes the experimental testing equipment and procedure used in determining temperatures at which liquid-liquid phase separation occurs for several different absorbent compositions and presents the results of the experiments.

The laboratory unit used to conduct the experiments included an absorber, a regenerator equipped with a steam supplied kettle-type reboiler, and associated pumps, exchangers and instrumentation. The sample point for the absorbent composition was located at the outlet from the over-flow section (sump section) of the kettle-type reboiler.

The kettle-type reboiler of the laboratory unit defined a heating zone. Provided within the heating zone was an internal weir that maintained on one side a level of liquid at the height of the internal weir. The internal weir, thus, provided for a liquid volume and for an overflow of the liquid into a sump section of the kettle-type reboiler on the opposite side of the internal weir. Liquid was withdrawn from the sump section for transfer and conveyance to a contact absorber. A heating coil capable of receiving and passing steam therethrough was provided that passed through the liquid volume that resided behind the internal weir. The kettle-type reboiler also was equipped with an outlet conduit that provided for the withdrawal of vapor from the heating zone and conveyance thereof to the regenerator of the laboratory unit.

The laboratory unit was operated such that the absorber pressure ranged from 55.16 to 79.29 kPa gauge (median of 59.98 kPa gauge) (8 to 11.5 psig (median of 8.7 psig)), the regenerator pressure ranged from 47.57 to 75.84 kPa gauge (median 64.81 kPa gauge) (6.9 to 11 psig (median of 9.4 psig)), and the lean solvent temperature to the absorber of approximately 70 °C while the solvent was being circulated through the system.

In the experimental runs of this Example 2 in which multiple liquid phases were formed in the liquid volume, it is believed that at least a light phase and a heavy phase were formed with the light phase residing above the heavy phase. The light phase would overflow the internal weir into the sump section of the kettle-type reboiler. This mechanism accounts for the different compositions of the liquid phases of the absorbent composition before and after the separation of the absorbent solution into the several liquid phases upon heating.

The compositions of the absorbent solutions and the results of the testing are presented in Table 2.

### Run No. 1 (Comparative)

Comparative Solution No. 1 (45% amine mixture, 55% water, no sulfolane) was placed in the laboratory unit and circulated. When the reboiler temperature reached 93 °C a sample was removed from the overflow internal weir compartment of the reboiler and titrated with a standard acid solution. The titration of the solution sampled from the overflow internal weir compartment consumed 22 ml of acid. The circulation of the solution continued until the reboiler temperature reached 113 °C. The titration of the solution sampled from the overflow internal weir compartment when the reboiler was at a temperature of 113 °C consumed 10 ml of acid. These data indicate that the solution, i.e., aqueous solvent comprising the amine mixture of the invention and water with an absence of an organic co-solvent such as sulfolane, separated into at least two liquid phases at a temperature greater than 93 °C and at or below 113 °C.

### Run No. 2 (Comparative)

Comparative Solution No. 2 (42.8% amine mixture, 52.4% water, 4.8 wt. % sulfolane) was placed in the laboratory unit and circulated. When the reboiler temperature reached 87 °C a sample was removed from the overflow internal weir compartment of the reboiler and titrated with a standard acid solution. The titration of the solution sampled from the overflow internal weir compartment consumed 21.7 ml of acid. The circulation of the solution continued until the reboiler temperature reached 120 °C. The titration of the solution sampled from the overflow internal weir compartment consumed 10.5 ml of acid. These data indicate that the solution phase-separated at a temperature greater than 87 °C and at a temperature at least or below 120 °C and that a 4.8 wt. % sulfolane was not sufficient to prevent phase separation of the solution.

### Run No. 3

Solution No. 3 (40.9% amine solution, 50 water, 9.1 wt. % sulfolane) was placed in the laboratory unit and circulated. During the circulation of the solution through the system, when the reboiler temperature was approximately 120 °C, samples were removed at periodic intervals from the overflow internal weir of the reboiler and titrated with a standard acid solution. The titration of the first sample of the solution, when the reboiler temperature was 120.8 °C, consumed 20.5 ml of acid. The titration of the solution samples taken after another 30 minutes, 41 minutes, 167 minutes, and 284 minutes, respectively, consumed 20 ml acid, 20.1 ml acid, 20 ml acid, and 19.9 ml acid.

These data indicate that the use of 9.1 wt. % sulfolane co-solvent in the solution prevented phase separation of the solution at a typical reboiler temperature of around 120 °C and that the prevention of the liquid-liquid phase separation was maintained over time.

### Run No. 4 (Comparative)

Comparative Solution No. 4 (42.3% amine solution, 51.7% water, 6 wt. % sulfolane) was placed in the laboratory unit and circulated. A sample of the solution was titrated with a standard acid solution when it was at room temperature, and it consumed 20 ml of acid. The solution was circulated through the system. When the reboiler temperature reached 113 °C a sample was taken from the overflow internal weir compartment of the reboiler and titrated with a standard acid solution. The titration of the solution sampled consumed 19.9 ml of acid. These data indicate that 6 wt. % sulfolane was sufficient to maintain the liquid phase of the solution in a single phase and to prevent liquid-liquid phase separation of the solution.

**Table 2. Absorbent compositions and titration results indicating the occurrence of phase separation at various reboiler temperatures.**

| Run No. | Amine Mixture | Water | Sulfolane | Titration of Liquid from Reboiler Sump when Liquid was at Reboiler Temp (1) | Reboiler Temp (1) | Titration of Liquid from Reboiler Sump when Liquid was at Reboiler Temp (2) | Reboiler Temp (2) |
|---|---|---|---|---|---|---|---|
| | weight units | weight units | weight units | | | | |
| | (wt. %) | (wt. %) | (wt. %) | (ml) | (°C) | (ml) | (°C) |
| 1 Comp. | 5850 (45 %) | 7150 (55 %) | 0 (0 %) | 22 | 93 | 10 | 113 |
| 2 Comp. | 5850 (42.8 %) | 7150 (52.4 %) | 650 (4.8 %) | 21.7 | 87 | 10.5 | 120 |
| 3 | 5850 (40.9 %) | 7150 (50.0 %) | 1300 (9.1 %) | 20.7 | 120 | 20 | 120 |
| 4 Comp. | 5850 (42.3 %) | 7150 (51.7 %) | 830 (6 %) | 20 | Room Temp | 19.9 | 113 |

The data presented above show that liquid-liquid phase separation of the absorbent composition within an absorption/regeneration system for the treatment of gas streams having a concentration of an acidic gas component occurs at typical reboiler operating temperatures. Also, the data show that the use or application of an organic co-solvent, such as the sulfone, sulfolane, can prevent phase separation of the amine mixture component of the absorbent composition that appears to occur at elevated temperatures. For certain aqueous solvents, which include the amine mixture of the invention and water as components, a sulfolane concentration in the range of from about 5 wt. % to about 10 wt. % provide for the miscibility of the components at the elevated temperatures and contribute to the inhibition of the phase separation of the components of the absorbent.

### Example 3

This Example describes the experimental testing equipment and procedure used in measuring certain selectivity properties of the inventive absorbent composition versus a comparison absorbent, N-methyl diethanolamine (MDEA), in the removal of H₂S relative to CO₂ from a gas stream containing H₂S and CO₂.

A stirred-cell absorption vessel was used to conduct the experiments. The reactor vessel was one liter glass reactor provided with liquid phase sample ports, adjustable stirring paddles for the vapor and liquid phases, thermal jacketing, a thermocouple port, a gas inlet and a gas outlet.

In conducting the experiment, the glass vessel was filled with 750 ml (at ambient temperature) of the absorbent composition (either the amine mixture of the invention or MDEA) leaving about 250 ml of vapor volume. The surface of the liquid was maintained as a quiet planar interface during the stirring of the vapor and liquid phases at a rate of 100 rpm. The temperature was maintained at approximately 25 °C.

The gas introduced into the inlet port of the vessel comprised 89 mole % nitrogen, 1 mole % H₂S and 10 mole % CO₂. The H₂S and CO₂ concentration of the outlet gas stream was monitored.

Presented in FIG. 2 are selected results from the testing.

FIG. 2 presents plots of the measured rate ratio of the H₂S absorption rate (mole H₂S/m²/sec) to the CO₂ absorption rate (mole CO₂/m²/sec) as a function of the H₂S concentration in the outlet gas for the amine mixture of the invention and for MDEA. As can be observed from the presented plots, the rate ratio for the amine mixture is consistently greater than the corresponding rate ratio for the MDEA. This indicates that the H₂S absorption selectivity of amine mixture is greater than the H₂S absorption selectivity of MDEA.

### Example 4

This Example presents the experimental results from testing the inventive amine mixture and a comparison solvent, MDEA, to determine the effect of CO₂ on H₂S slip from an absorber and the effect of CO₂ on the percent CO₂ absorption.

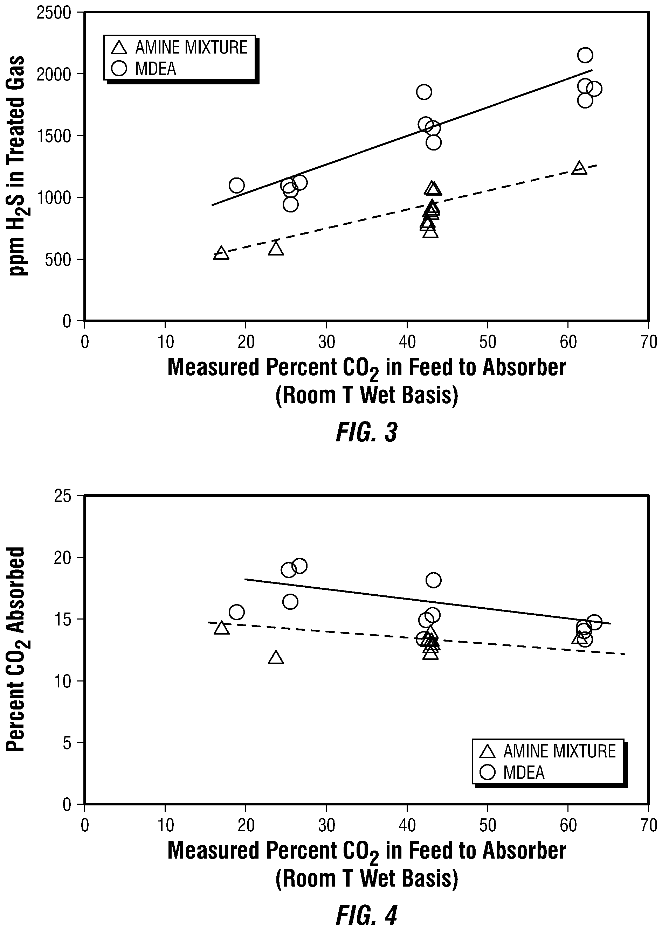

The laboratory unit described in Example 2 was used to conduct the experiments of this Example 4. Certain of the results from these experiments are presented in FIG. 3 and FIG. 4. The gas feed charged to the absorber comprised H₂S at a targeted concentration of from 0.6 to 0.7 mole %. The CO₂ concentration of the gas feed was that as expressed along the abscissa (x) axis of the plots of FIG. 3 and FIG. 4, and the balance of the gas feed was N₂ gas.

FIG. 3 graphically presents the measured H₂S concentration in the treated outlet gas from the reactor vessel as a function of the CO₂ contained in the inlet gas to the reactor vessel for the amine mixture of the invention and MDEA. As may be observed from the data presented, the amine mixture provides for a significantly lower H₂S concentration in the treated gas for a given CO₂ concentration in the inlet gas to the reactor vessel. This indicates that the amine mixture provides for a much greater H₂S removal than does the MDEA for all levels of CO₂ concentration of a gas to be treated.

FIG. 4 graphically presents the measured percentage of the CO₂ that is contained in an inlet gas to the reactor vessel that is removed by absorption with the amine mixture and with MDEA as a function of the concentration of CO₂ in the inlet gas to the reactor vessel. These data indicate that the amine mixture is less effective in absorbing CO₂ from a gas stream than is MDEA. This is a good characteristic for the amine mixture; since, a higher selectivity in the absorption of H₂S relative to the absorption of CO₂ is desired.

## Claims

1. A method of improving a process for the selective removal of hydrogen sulfide from a gas stream, comprising hydrogen sulfide and carbon dioxide, wherein said process utilizes a system for treating said gas stream, which said system contains an inventory of an amine absorbent composition that includes an H₂S lean absorbent composition and an H₂S rich absorbent composition and said system includes a contacting column for contacting said H₂S lean absorbent composition with said gas stream to yield a treated gas stream and said H₂S rich absorbent composition and a regenerator system for receiving and regenerating said H₂S rich absorbent composition to yield said H₂S lean absorbent composition, wherein said method comprises:
o providing said amine absorbent composition, wherein said amine absorbent composition comprises:
• from 75 wt. % to 92 wt.%, based on the total weight of said absorbent composition, of an aqueous solvent, said aqueous solvent comprising from 20 wt. % to 70 wt. %, based on the total weight of said aqueous solvent, of an amination reaction product of a polydispersed polyethylene glycol (PEG) mixture having an average molecular weight that is in the range of from 180 to 1000 and t-butylamine, and from 30 wt. % to 80 wt. % water, based on the total weight of said aqueous solvent,
- wherein said PEG mixture comprises polyethylene glycols of the formula HOCH2(CH2OCH2)nCH2OH, wherein n is an integer selected from values in the range of from 1 to 24, and
- wherein said PEG mixture includes two or more different PEG compounds having the aforementioned formula, wherein n is an integer selected from values in the range of from 1 to 24, and
- wherein said amination reaction product comprises at least a first sterically hindered amine and a second sterically hindered amine, and
- wherein the amination reaction product has been prepared using an amination catalyst comprising: from 40 to 90 wt. % nickel; from 4 to 40 wt. % copper; and from 1 to 50 wt. % of either zirconium or chromium, or a combination of both zirconium and chromium, and
- wherein said first sterically hindered amine is selected from the group of amine compounds of the formula: (CH3)3CNH(CH2CH2O)xCH2CH2NHC(CH3)3, wherein x is an integer in the range of from 3 to 14; and
- wherein said second sterically hindered amine is selected from the group of amine compounds having the following formula: (CH3)3CNH(CH2CH2O)xCH2CH2OH, wherein x is an integer in the range of from 3 to 14,
- wherein the weight ratio of the first sterically hindered amine to the second sterically hindered amine is in the range of from 3:1 to 6:1; and
• from 8 wt. % to 25 wt. %, based on the total weight of said absorbent composition, of an organic co-solvent, wherein said organic co-solvent is selected from the group consisting of sulfones, sulfone derivatives, and sulfoxides.

2. A process as recited in claim 1, wherein the amination catalyst further comprises from 0.2 to 20 wt. % tin.

3. A method as recited in claim 1, wherein said organic co-solvent is either a substituted or unsubstituted cyclotetramethylene sulfone, wherein no more than two alkyl substituents are appended to the tetramethylene sulfone ring and the alkyl substituents have from 1 to 4 carbon atoms.

4. A method as recited in claim 1, wherein said aqueous solvent is present in said absorbent composition in an amount in the range of from 85 wt. % to 92 wt. %, and wherein said organic co-solvent present in said absorbent composition is in an amount in the range of from 8 wt. % to 15 wt. %.

## Patentansprüche

1. Verfahren zum Verbessern eines Verfahrens zur selektiven Entfernung von Schwefelwasserstoff aus einem Gasstrom umfassend Schwefelwasserstoff und Kohlendioxid, wobei das Verfahren ein System zum Behandeln des Gasstroms verwendet, wobei das System ein Inventar einer aminabsorbierenden Zusammensetzung enthält, das eine H₂S-abgereicherte absorbierende Zusammensetzung und eine H₂S-angereicherte absorbierende Zusammensetzung einschließt, und das System eine Kontaktkolonne zum Inkontaktbringen der H₂S-abgereicherten absorbierenden Zusammensetzung mit dem Gasstrom, um einen behandelten Gasstrom zu erhalten, und der H₂S-angereicherten absorbierenden Zusammensetzung und einem Regeneratorsystem zum Aufnehmen und Regenerieren der H₂S-angereicherten absorbierenden Zusammensetzung zum Erhalten der H₂S-abgereicherten absorbierenden Zusammensetzung einschließt, wobei das Verfahren Folgendes umfasst:
o Bereitstellen der aminabsorbierenden Zusammensetzung, wobei die aminabsorbierende Zusammensetzung umfasst:
• 75 Gew.-% bis 92 Gew.-%, bezogen auf das Gesamtgewicht der absorbierenden Zusammensetzung, eines wässrigen Lösungsmittels, wobei das wässrige Lösungsmittel 20 Gew.-% bis 70 Gew.-%, bezogen auf das Gesamtgewicht des wässrigen Lösungsmittels, eines Aminierungsreaktionsprodukts einer polydispersen Polyethylenglycol(PEG)-Mischung mit einer durchschnittlichen Molekülmasse im Bereich von 180 bis 1000 und t-Butylamin und 30 Gew.-% bis 80 Gew.-% Wasser, bezogen auf das Gesamtgewicht des wässrigen Lösungsmittels, umfasst,
- wobei die PEG-Mischung Polyethylenglykole der Formel HOCH₂(CH₂OCH₂)ₙCH₂OH umfasst, wobei n eine ganze Zahl ist, die aus Werten im Bereich von 1 bis 24 ausgewählt ist, und
- wobei die PEG-Mischung zwei oder mehr verschiedene PEG-Verbindungen enthält, die die vorstehend genannte Formel aufweisen, wobei n eine ganze Zahl ist, die aus Werten im Bereich von 1 bis 24 ausgewählt ist, und
- wobei das Aminierungsreaktionsprodukt mindestens ein erstes sterisch gehindertes Amin und ein zweites sterisch gehindertes Amin umfasst, und
- wobei das Aminierungsreaktionsprodukt unter Verwendung eines Aminierungskatalysators hergestellt wurde, umfassend: 40 bis 90 Gew.-% Nickel; 4 bis 40 Gew.-% Kupfer; und 1 bis 50 Gew.-% von entweder Zirkonium oder Chrom oder eine Kombination von sowohl Zirkonium als auch Chrom und
- wobei das erste sterisch gehinderte Amin ausgewählt ist aus der Gruppe von Aminverbindungen der Formel: (CH₃)₃CNH(CH₂CH₂O)ₓCH₂CH₂NHC(CH₃)₃, wobei x eine ganze Zahl im Bereich von 3 bis 14 ist; und
- wobei das zweite sterisch gehinderte Amin ausgewählt ist aus der Gruppe von Aminverbindungen der folgenden Formel: (CH₃)₃CNH(CH₂CH₂O)ₓCH₂CH₂OH, wobei x eine ganze Zahl im Bereich von 3 bis 14 ist; und
- wobei das Gewichtsverhältnis des ersten sterisch gehinderten Amins zu dem zweiten sterisch gehinderten Amin im Bereich von 3:1 bis 6:1 liegt; und
• 8 Gew.-% bis 25 Gew.-%, bezogen auf das Gesamtgewicht der absorbierenden Zusammensetzung, eines organischen Co-Lösungsmittels, wobei das organische Co-Lösungsmittel ausgewählt ist aus der Gruppe bestehend aus Sulfonen, Sulfonderivaten und Sulfoxiden.

2. Verfahren nach Anspruch 1, wobei der Aminierungskatalysator ferner 0,2 bis 20 Gew.-% Zinn umfasst.

3. Verfahren nach Anspruch 1, wobei das organische Co-Lösungsmittel entweder ein substituiertes oder ein unsubstituiertes Cyclotetramethylensulfon ist, wobei an dem Tetramethylsulfonring nicht mehr als zwei Alkylsubstituenten angehängt sind und die Alkylsubstituenten 1 bis 4 Kohlenstoffatome aufweisen.

4. Verfahren nach Anspruch 1, wobei das wässrige Lösungsmittel in der absorbierenden Zusammensetzung in einer Menge im Bereich von 85 Gew.-% bis 92 Gew.-% vorliegt, und wobei das organische Co-Lösungsmittel in der absorbierenden Zusammensetzung in einer Menge im Bereich von 8 Gew.-% bis 15 Gew.-% vorliegt.

## Revendications

1. Procédé d'amélioration d'un procédé d'élimination sélective de sulfure d'hydrogène d'un courant gazeux, comprenant du sulfure d'hydrogène et du dioxyde de carbone, ledit procédé utilisant un système de traitement dudit courant gazeux, ledit système contenant un inventaire d'une composition absorbant les amines qui comprend une composition absorbante pauvre en H₂S et une composition absorbante riche en H₂S et ledit système comprenant une colonne de mise en contact pour mettre en contact ladite composition absorbante pauvre en H₂S avec ledit courant gazeux afin de produire un courant de gaz traité et ladite composition absorbante riche en H₂S et un système régénérateur pour recevoir et régénérer ladite composition absorbante riche en H₂S afin d'obtenir ladite composition absorbante pauvre en H₂S, dans lequel ledit procédé comprend :
o la fourniture de ladite composition absorbante d'amines, ladite composition absorbante d'amines comprenant:
• de 75 % à 92 % en poids, par rapport au poids total de ladite composition absorbante, d'un solvant aqueux, ledit solvant aqueux comprenant de 20 % à 70 % en poids, par rapport au poids total dudit solvant aqueux, d'un produit de réaction d'amination d'un mélange de polyéthylène glycol polydispersé (PEG) ayant un poids moléculaire moyen compris entre 180 et 1 000 et de t-butylamine, et compris entre 30 et 80 % en poids en eau, par rapport au poids total dudit solvant aqueux,
- dans lequel ledit mélange de PEG comprend des polyéthylène glycols de formule HOCH₂(CH₂OCH₂)ₙCH₂OH, dans laquelle n est un entier choisi parmi des valeurs comprises entre 1 et 24, et
- dans lequel ledit mélange de PEG comprend deux ou plusieurs composés PEG différents ayant la formule susmentionnée, dans laquelle n est un entier choisi parmi les valeurs comprises entre 1 et 24, et
- dans lequel ledit produit de réaction d'amination comprend au moins une première amine à encombrement stérique et une deuxième amine à encombrement stérique, et
- dans lequel le produit de réaction d'amination a été préparé en utilisant un catalyseur d'amination comprenant : de 40 à 90 % en poids de nickel ; de 4 à 40 % en poids de cuivre ; et de 1 à 50 % en poids de zirconium ou de chrome, ou d'une combinaison de zirconium et de chrome, et
- dans lequel ladite première amine à encombrement stérique est choisie dans le groupe des composés amines de formule : (CH₃)₃CNH(CH₂CH₂O)ₓCH₂CH₂NHC(CH₃)₃, dans laquelle x est un entier compris entre 3 et 14 ; et
- dans lequel ladite seconde amine à encombrement stérique est choisie dans le groupe des composés amines répondant à la formule suivante : (CH₃)₃CNH (CH₂CH₂O)ₓCH₂CH₂OH, dans laquelle x est un entier compris entre 3 et 14,
- dans lequel le rapport pondéral de la première amine à encombrement stérique à la deuxième amine à encombrement stérique est compris dans l'intervalle de 3:1 à 6:1 ; et
• de 8 % à 25 % en poids, par rapport au poids total de ladite composition absorbante, d'un co-solvant organique, ledit co-solvant organique étant choisi dans le groupe constitué de sulfones, de dérivés de sulfone et de sulfoxydes.

2. Procédé selon la revendication 1, dans lequel le catalyseur d'amination comprend en outre de 0,2 à 20 % en poids d'étain.

3. Procédé selon la revendication 1, dans lequel ledit co-solvant organique est une cyclotétraméthylène sulfone substituée ou non substituée, dans lequel pas plus de deux substituants alkyles sont ajoutés au cycle tétraméthylène sulfone et les substituants alkyles ont de 1 à 4 atomes de carbone.

4. Procédé selon la revendication 1, dans lequel ledit solvant aqueux est présent dans ladite composition absorbante en une quantité dans la plage de 85 % à 92 % en poids et dans lequel ledit co-solvant organique présent dans ladite composition absorbante représente une quantité dans la plage de 8 % à 15 % en poids.
